(19) Europäisches Patentamt
European Patent Office
Office européen des brevets

(11) Publication number: **0 540 644 B1**

(12) # EUROPEAN PATENT SPECIFICATION

(45) Date of publication of patent specification: **23.08.95**

(51) Int. Cl.[6]: **C08G 59/44**

(21) Application number: **91914253.9**

(22) Date of filing: **24.06.91**

(86) International application number:
**PCT/US91/04526**

(87) International publication number:
**WO 92/01726 (06.02.92 92/04)**

The file contains technical information submitted after the application was filed and not included in this specification

(54) **SUBSTITUTED CYANOGUANIDINES AS CURING AGENTS FOR EPOXY RESINS.**

(30) Priority: **23.07.90 US 557445**

(43) Date of publication of application:
**12.05.93 Bulletin 93/19**

(45) Publication of the grant of the patent:
**23.08.95 Bulletin 95/34**

(84) Designated Contracting States:
**AT BE CH DE DK ES FR GB GR IT LI LU NL SE**

(56) References cited:
**EP-A- 0 306 451**
**US-A- 3 553 166**
**US-A- 4 581 422**
**US-A- 4 990 232**

(73) Proprietor: **AlliedSignal Inc.**
**101 Columbia Road,**
**P.O. Box 2245**
**Morristown,**
**New Jersey 07962-2245 (US)**

(72) Inventor: **ZUPANCIC, Joseph, James**
**17W 338 Belmont Avenue**
**Bensenville, IL 60106 (US)**
Inventor: **CONRAD, Jeffrey, Paul**
**3418 North Lakewood, Apt. 1S**
**Chicago, IL 60657 (US)**

(74) Representative: **Brock, Peter William et al**
**Urquhart-Dykes & Lord**
**1 Richfield Place**
**Richfield Avenue**
**Reading RG1 8EO**
**Berkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 540 644 B1

## Description

The invention relates to the curing of epoxy resins and, more particularly, to improvement of the curing agents used with epoxy resins. Dicyanodiamide (also called cyanoguanidine) is well-known as a curing agent for epoxy resins and replacing it with an improved curing agent has been an objective of the present inventors.

Dicyanodiamide is well-known as a curing agent for epoxy resins but it is also known to have a serious deficiency. It is only soluble in solvents which are undesirable, either because they are not usable in most applications, such as water, or the solvents are relatively expensive and environmentally undesirable, such as dimethylformamide and the like.

In US-A-3,553,166 a curing agent is disclosed which combines a metal salt of an imidazole and another compound, which may be dicyanodiamide. These curing agents are used in a one-part epoxy composition which is curable at elevated temperatures, but which can be stored at ambient temperature for long periods.

Another combination of dicyanodiamide with a second compound is found in US-A-4,311,753 where dicyanodiamide is combined with tetraalkyl guanidine to provide a curing agent for mixtures of di- and tetra-functional epoxides.

The reaction product of dicyanodiamide with formaldehyde and an amine terminated polyether may be used to cure epoxy resins, as is shown in US-A-4,581,422. Such a product is a substituted cyanoguanidine, but by adding an amine terminated polyether and a methylene group from the formaldehyde it differs from those of the present inventors, as will be seen below.

A somewhat similar composition is disclosed in JP-A-61-207425 which employs an epoxy hardener combining cyanoguanidine compounds, polyetheramines, and guanide compounds.

Two recent published European patent applications cover certain substituted cyanoguanidines said to be useful as curing agents for epoxy resins. In EP-A-306,451 oligomers of substituted 3-cyanoguanidines are discussed. Such compounds are produced by the reaction of a monoisocyanate with a diisocyanate to form the oligomer, followed by reaction with cyanamide to form compounds having the formula:

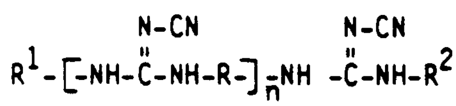

These compounds differ from those of the present inventors in that they are oligomers and both the terminal amino groups are substituted, thus reducing the functionality of these compounds. The applicants recognized the problem discussed above which is inherent with the use of dicyanodiamide, namely, the need to use objectionable solvents. The new oligomeric cyanoguanidines are said to dissolve well in suitable solvents and to produce epoxy resins having a high glass transition temperature. Another published application is EP-A-310,545 in which di-substituted cyanoguanidines having the formula:

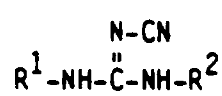

are said to be useful as curing agents for epoxy resins. These substituted cyanoguanidines are prepared by reacting a di-substituted carbodiimide ($R^1$-N = C = N-$R^2$) with cyanamide ($NH_2$C = N). As with the compounds of EP-A-306,451, these di-substituted cyanoguanidines are substituted at both the amine groups.

Substituted cyanoguanidines have also been suggested as being useful in the preparation of polyurethanes as shown in US-A-3 734 868.

Related materials have also been used as precursors to various pharmaceuticals, an example of which is US-A-4 560 690.

US-A-2 455 807 is relevant to the preferred method of preparing the substituted cyanoguanidine compounds of the present invention. The reaction of dicyanamide with the desired substituted amine is shown to produce the substituted cyanoguanidine.

The present invention refers to a composition comprising an epoxy resin and a curing agent for said epoxy resin which is a mono-substituted cyanoguanidines having the formula:

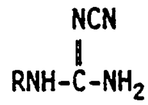

where R is $-CH_2C_6H_4X$, or $-CH_2CH_2C_6H_4X$,
and X is either -H, $-CH_3$, $-OCH_3$, -OH, or $-NY_2$
and Y is -H or $-CH_3$
Such mono-substituted cyanoguanidines are soluble in various solvents such as acetone, methyl ethyl ketone, methyl isobutyl ketone, and cyclohexanone. In service as curing agents the substituted cyanoguanidines generally will be employed in amounts up to 20 wt.% of the epoxy resin, preferably 4 to 10 wt.%.

In another aspect, the invention is a method for curing epoxy resins in which an effective amount of a substituted cyanoguanidine as defined above is added to an epoxy resin, preferably in amounts up to 20 wt.%, more preferably 4 to 10 wt.%, and the curing carried out under curing conditions.

It has been recognized that dicyanodiamide (i.e. cyanoguanidine), although widely used, is an undesirable curing agent for epoxy resins, since it requires the use of objectionable solvents such as dimethylformamide and the like. If more soluble compounds could be found which provide equivalent or improved curing properties, then cyanoguanidine could be replaced and the undesirable solvents avoided. Reduced production costs from the use of less expensive solvents also could be the result of such a change in curing agents. The present inventors have investigated potential curing agents and have found a group of substituted cyanoguanidines which have significant advantages over the parent compound.

Composition of Substituted Cyanoguanidines

Mere substitution of other groups for the hydrogen atoms in cyanoguanidine is not sufficient to provide the desired results. As will be seen, many substituted cyanoguanidines do not have the improved solubility characteristics sought. Further, substitution of both of the amino groups of cyanoguanidine that is, compounds of the generic formula:

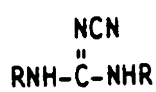

such as are shown in EP-A-310,545 are less satisfactory, as will be shown below.

The inventors have found that mono-substituted cyanoguanidines having the formula

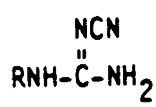

where R is $-CH_2C_6H_4X$, or $-CH_2CH_2C_6H_4X$,
and X is either -H, $-CH_3$, $-OCH_3$, -OH, or $-NY_2$
and Y is -H or $-CH_3$
have improved solubility over the parent compound and still retain a high curing ability so that they are effective in amounts of up to 20 wt. percent, preferably 4 to 10 wt. percent, of the epoxy resin.

The mono-substituted cyanoguanidines of this invention and the di-substituted cyanoguanidines of EP-A-310 545 differ in the degree of reactive functionality. In order for the curing agent to react and generate a cross-linked epoxy based polymer network, the amine radicals react with the epoxide radicals. The degree of reactive functionality of the cyanoguanidine will depend on the degree of functionalization for the cyanoguanidine, which is related to the number of exchangeable nitrogen hydrogens of the cyanoguanidine. For example, dicyanodiamide (cyanoguanidine) has a defined degree of functionality of four, that is, it is capable of addition to four epoxide radicals. In the case of a monosubstituted cyanoguanidine the degree of functionality is three and it is capable of reacting with three epoxide radicals. For a di-substituted cyanoguanidine as in EP-A-310 545 the degree of functionality is two, and it is capable of reacting with two epoxide radicals.

The degree of reactive functionality for the curing agent will affect the type of polymer network formed in the cured polymer system and consequently will affect the performance properties in B-Stage or a prepreg, such as the viscosity as a function of cure, the solvent resistance for the polymer, the glass transition temperature (Tg) for the polymer, and the coefficient of thermal expansion ($\alpha_g$). For example, when a curing agent with a degree of reactive functionality of two (EP-A-310 545) is employed, the network will have a high degree of linear structures with only a minor degree of branching. When a curing agent with a degree of reactive functionality of three (the present invention) is employed, the polymer network will have a high degree of branched or star-like structures and a minimum of linear type structures. The branched or star-like structures will affect the resin flow properties (resin flow viscosity) for the B-Staged resin during lamination. If the resin flow viscosity is low then the laminates or composites formed will experience a high degree of resin flow, generating laminates or composites with voids or resin-poor products. Linear structures in the cured polymer will yield poor solvent resistance due to the solvation of the polymer fragments or swelling of the polymer. Branched or star-like structures provide improved solvent resistance due to the formation of a more highly cross-linked network. Linear structures will yield a polymer of lower Tg and may yield higher coefficient of thermal expansion than a polymer with branched structures.

## Solvents

The parent compound, cyanoguanidine, is soluble only in a small group of generally available solvents, including dimethylformamide, dimethylsulfoxide, dimethylacetamide, N-methyl-2-pyrrolidinone and methanol. The mono-substituted cyanoguanidines of the invention are soluble in a number of more desirable solvents, including acetone, methyl ethyl ketone, methyl isobutyl ketone, cyclohexanone, methanol, ethanol, or neat epoxy resins. In particular, acetone, methyl ethyl ketone, methyl isobutyl ketone, and cyclohexanone are preferred solvents.

The compounds of the invention are generally soluble in amounts up to about 45 wt.% in such solvents. In many cases the desired amount of the substituted cyanoguanidine is dissolved in enough solvent to make a solution containing about 13 to 45 wt.% of the compound and thereafter the solution is mixed with the epoxy precursors and cured under curing conditions.

## Epoxy Resins

The substituted cyanoguanidines of the invention may be used with various epoxy resin precursors known in the art. In general, these will include diglycidyl bisphenol-A (DGEBA), diglycidyl tetrabromobisphenol-A, triglycidyl triphenol methane, triglycidyl triphenol ethane, tetraglycidyl tetraphenolethane, tetraglycidyl methylene dianiline and oligomers and mixtures thereof. More particularly, diglycidyl bisphenol-A (DGEBA) types have been found to provide suitable results with the curing agents of the invention.

## Catalyst for Epoxy Resins

In order to facilitate the reaction of the substituted cyanoguanidine with the epoxy resin it may prove useful to employ a catalyst. There are various catalyst known in the art which can be employed in this invention. The catalyst which we believe offer distinct advantages over others will be free of transition metals. In general, the catalyst will include imidazole, 2-methylimidazole, 2-phenylimidazole, 1-cyanoethyl-2-phenylimidazole, 1-(2-cyanoethyl)-2-ethyl-4-methylimidazole, 4-phenylimidazole, 2-ethylimidazole, 2-ethyl-4-methylimidazole, benzyldimethylamine, 4-(dimethylamino)-N,N-dimethylbenzylamine, 4-methoxy-N,N-dimethylbenzylamine, 4-methyl-N,N-dimethylbenzylamine, and the like.

## Preparation of Laminates

An advantage of the substituted cyanoguanidines of the invention is their ability to replace the cyanoguanidine (dicyanodiamide) commonly used to cross-link epoxy resins in the preparation of reinforced laminates for the electronics industry and without requiring the use of undesirable solvents. The methods used to prepare such laminates are well known to those skilled in the art and need not be discussed in detail here in connection with the present invention since the procedures are not revised to accommodate the substituted cyanoguanidines of the invention. In general, It may be stated that the fabric which is to be used to reinforce the laminate, typically made of glass fibers, is coated with epoxy resins combined with the crosslinking agents and a catalyst as desired. The coated fabric is then heated in order to drive off solvents

and to cure (polymerize and crosslink) the epoxy resins and the crosslinking agents. Multiple layers of coated fabric are commonly combined to provide the laminates needed for electronic circuit boards. When only a partial cure is carried out, the resulting product is often referred to as a "prepreg" or "B-stage" material. Further curing is later carried out to complete the laminate. These processes are carried out in batch or continuous processes familiar to those skilled in the art.

Preparation of the Substituted Cyanoguanidines

The curing agents of the invention may be prepared by various methods known to those skilled in the art. For example, the method of May (J. Org. Chem., 12, 437-442, 442-445 (1947)) and Curd (J. Chem. Soc., 1630-1636 (1948)) reaction of an aryl isothiocyanate with sodium cyanamide, followed by reaction with methyl iodide to generate a N-cyano-S-methyl-N'-arylisothiourea which upon reaction with ammonia yields a substituted cyanoguanidine. Another method of Curd (J. Chem. Soc., 729-737 (1946))) and Rose (GB-A-577,843) employs the reaction of an aryl diazonium salt with dicyanodiamide to yield a substituted aryl-azo-dicyanodiamide or triazene which thermally decomposes to yield nitrogen and the corresponding substituted cyanoguanidine.

The inventors consider the method in US-A-2,455,807 to be of particular value in preparing their substituted cyanoguanidines. This method may be described generically by the following reaction according to Redmon et al.

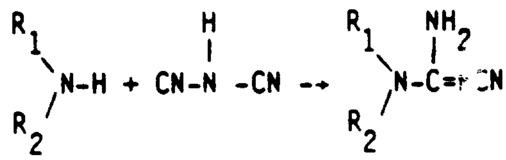

In practice, a metal salt of dicyanamide (CN-NH-CN) preferably is used, such as sodium dicyanamide. The selected substituted amine, for example, benzyl amine may be dissolved in a suitable solvent, such as butanol, ethanol, propanol and water, or mixed with hydrochloric acid to form a slurry. Sodium dicyanamide is added in an approximately stoichiometric quantity. The reaction is carried out at temperatures between about 75° and 110°C and at pressures of atmospheric to 2068 kPa for a period of time necessary to complete the reaction. Preferably, a temperature of about 100° to 110°C will be used with the reaction time being about 2 to 24 hours. Thereafter, if used, the solvent is distilled off and the substituted cyanoguanidine is recovered by crystallization and washing. If hydrochloric acid is used, the solids are filtered, washed, and then redissolved and crystallized from solution.

EXAMPLE 1

Synthesis of N-Benzyl-dicyanodiamide (BZDICY): 196.20 g (1.830 moles) of benzylamine and ca. 1560 mL of butanol are charged into a 5 liter 3-neck round bottom flask equipped with condenser, addition funnel and mechanical stirrer. To this stirred reaction mixture is added 100.56 g (55.0 mL, 1.020 moles) of concentrated sulfuric acid in 400 mL of butanol dropwise, final pH is 7 to 8. To the above reaction mixture is added 182.57 g (2.050 moles) of sodium dicyanamide and 40 mL of water, the reaction mixture is heated with stirring to 100°C and maintained at 100°C ± 5°C for 6.5 hrs, during the course of the reflux 95 mL of water is added to the reaction. To the cooled reaction mixture is added an excess of water (3 liters), the butanol is azeotropically distilled off. Upon cooling a precipitate separates from the oil, which is filtered off and then washed with 1000 mL of 5% sodium hydroxide solution, filtered, washed with 1000 mL of 5% acetic acid solution, filtered and washed with 1000 mL of water. The white crystalline solid is then recrystallized from boiling water, yielding 195.8 g (61.4% yield) of white crystalline product, m.p. 109-110°C (Lit. m.p. 108-109); Elemental Analysis, Found C 61.99%, H 6.08%, N 30.57%, Calculated C 62.04%, H 5.80%, N 32.16%.

EXAMPLE 2

Synthesis of N-Benzyl-Dicyanodiamide (BZDICY): 490.5 g (500 mL, 4.58 moles) of benzylamine is charged into a 5 liter 3-neck round bottom flask equipped with condenser, addition funnel and mechanical stirrer. To the stirred, chilled (2-5°C) benzylamine is added 448.9 g (378 mL, 4.58 moles) of concentrated

hydrochloric acid, at a rate that insures that the reaction mixture does not exceed 50 °C. The reaction slurry is heated to 75 °C and then 428.2 g (4.81 moles) of sodium dicyanamide is added and the reaction mixture is heated with stirring to 100 °C and maintained at 100 °C ±5 °C for 6.5 hrs. Add 1.5 liters of water to the stirred reaction mixture and allow to cool overnight. The reaction mixture is filtered and then rinsed with 1.5 liter of water. The crystalline product is stirred with 1.77 liters of 5% sodium hydroxide solution for 2.0 hrs, filtered, rinsed with 1.77 liter of water, stirred with 1.77 liters of 5% acetic acid solution, filtered and washed with 2.5 liters of water. The white crystalline solid is then dissolved in 1.6 liter of methanol and filtered to remove any insoluble material, the methanol solution is added to 8.0 liters of water and then filtered, yielding 472.9 g (59.7% yield) of white crystalline product, m.p. 107-109 °C.

EXAMPLE 3

Synthesis of N-(4-Methylbenzyl)-dicyanodiamide (MBZDICY): 200.00 g (1.650 moles) of 4-methylbenzylamine and ca. 1400 mL of butanol are charged into a 5 liter 3-neck round bottom flask equipped with condenser, addition funnel and mechanical stirrer. To this stirred reaction mixture is added 90.65 g (50.0 mL, 0.924 moles) of concentrated sulfuric acid in 370 mL of butanol dropwise. To the above reaction mixture is added 164.57 g (1.850 moles) of sodium dicyanamide and 37 mL of water, the reaction mixture is heated with stirring to 100 °C and maintained at 100 °C ±5 °C for 4.75 hrs, during the course of the reflux 85.00 mL of water is added to the reaction. To the cooled reaction mixture is added an excess of water (3 liters), the butanol is azeotropically distilled off. Upon cooling a precipitate separates from the oil, which is filtered off and then washed with 1000 mL of 5% sodium hydroxide solution, filtered, washed with 1000 mL of 5% acetic acid solution, filtered and washed with 1000 mL of water. The white crystalline solid is then recrystallized from boiling water, yielding 168.3 g (54.1% yield) of white crystalline product, m.p. 137-138 °C; Elemental Analysis, Found C 62.84%, H 6.26%, N 29.16%, Calculated C 63.79%, H 6.44%, N 29.77%.

EXAMPLE 4

Synthesis of N-(4-Methoxybenzyl)-dicyanodiamide (MOBZDICY): 200.00 g (1.460 moles) of 4-methoxybenzylamine and ca. 1230 mL of butanol are charged into a 5 liter 3-neck round bottom flask equipped with condenser, addition funnel and mechanical stirrer. To this stirred reaction mixture is added 80.08 g (43.5 mL, 0.816 moles) of concentrated sulfuric acid in 330 mL of butanol dropwise. To the above reaction mixture is added 145.38 g (1.630 moles) of sodium dicyanamide and 32 mL of water, the reaction mixture is heated with stirring to 100 °C and maintained at 100 °C ±5 °C for 6.0 hrs., during the course of the reflux 76 mL of water is added to the reaction. To the cooled reaction mixture is added an excess of water (3 liters), the butanol is azeotropically distilled off. Upon cooling a precipitate separates from the oil, which is filtered off and then washed with 1000 mL of 5% sodium hydroxide solution, filtered, washed with 1000 mL of 5% acetic acid solution, filtered and washed with 1000 mL of water. The white crystalline solid is then recrystallized from boiling water, yielding 103.8 g (34.8% yield) of white crystalline product, m.p. 91-93%C; Elemental Analysis, Found C 58.62%, H 6.06%, N 26.58%, Calculated C 58.80%, H 5.93%, N 27.44%.

EXAMPLE 5

Synthesis of 4-(N,N-Dimethylamino)benzylamine: 74.62 g (0.500 mols) of N,N-dimethylaminobenzaldehyde, 6.1815 g of Raney Nickel, and 200 mL of ethanol were charged into a glass liner for an 850 cc autoclave. The glass liner is placed within an autoclave and then 10.0 g of ammonia and 850 psi (58,6 bar) hydrogen at 25 °C. The reactor is then heated to 70 °C over a 1.5 hour period and maintained at 70 °C for 5 hrs. The reaction mixture is then allowed to cool to ambient temperature. The reaction mixture is filtered through a fritted glass filter to remove the catalyst; and the filtrate is concentrated under vacuum to yield a yellow liquid (crude yield 73.53 g, 97.9%)

Three comparable reaction runs were combined (crude yields of 73.53 g, 88.60 g. and 76.93 g) and vacuum distilled to yield a colorless liquid of 183.0 g (81.2%), b.p. 125 °C/2.4 mm (3,2 mbar) (Lit. b.p. 158-159 °C/25 mm (33,3 mbar)).

Synthesis of N'-4-(N,N-Dimethylamino)benzyl)-dicyanodiamide (DMABZDICY): 61.4 g (0.408 moles) of 4-(N,N-dimethylamino)benzylamine and Ca. 310 mL of butanol are charged into a 2 liter 3-neck round bottom flask equipped with condenser, addition funnel and mechanical stirrer. To this stirred reaction mixture is added 22.43 g (12.2 mL, 0.229 moles) of concentrated sulfuric acid in 90 mL of butanol dropwise. To the above reaction mixture is added 40.72 g (0.457 moles) of sodium dicyanamide and 10 mL of water,

the reaction mixture is heated with stirring to 100 °C and maintained at 100 °C ±5 °C for 5.5 hrs., during the course of the reflux 20 mL of water is added to the reaction. To the cooled reaction mixture is added an excess of water (1 liter), the butanol is azeotropically distilled off. Upon cooling a precipitate separates from the oil, which is filtered off and then washed with 300 mL of 5% sodium hydroxide solution, filtered, washed with 300 mL of 5% acetic acid solution, filtered and washed with 500 mL of water. The crystalline solid is then recrystallized from ethanol: water (90:10), yielding 55.2g (62.3% yield) of gray crystalline product, m.p. 137-139 °C; Elemental Analysis, Found C 61.41%, H 7.29%, N 32.34%, Calculated C 60.79%, H 6.97%, N 32.24%.

EXAMPLE 6

Synthesis of N-(4-hydroxybenzyl)-dicyanodiamide (HBZDICY): 36.45 g of 4-hydroxybenzylamine sulfate (0.106 mol) is slurried with 125 mL of butanol in a 500 mL 3-necked round bottom flask fitted with a Dean-Stark trap and a heating mantle. The mixture is neutralized with dilute aqueous sodium hydroxide. 91.9 g of sodium dicyanamide is then added with stirring and the temperature raised to 105 °C. The temperature is maintained at 105 °C for 4.75 hours. No water is collected in the trap. 300 mL of water is then added and the butanol is distilled azeotropically. The mixture is cooled and 200 mL of water are added. A resinous product precipitates. The water is decanted and the product dissolved in methyl isobutyl ketone (MIBK) and washed twice with water. The organic phase is dried over sodium sulfate, filtered and rotary evaporated leaving 16 g (79% of theoretical) of a brown resin.

EXAMPLE 7

Benzyl dicyanodiamide was tested for its solubility in various potential solvents at room temperatures. Increments of 0.5 g each were added to 10 g of the selected solvent until after 10-15 minutes of vigorous stirring the last increment failed to fully dissolve. The results are reported in the following table.

TABLE A

| Solvent | Solubility, g/g |
|---|---|
| propylene glycol methyl ether | 0.35 |
| Isopropanol | 0.15 |
| s-butanol | 0.2 |
| n-propanol | 0.15 |
| acetone | 0.65 |
| methyl ethyl ketone | 0.6 |
| ethyl acetate | 0.1 |
| n-butyl acetate | <0.05 |
| toluene | <0.05 |
| ethylene glycol | 0.35 |
| propylene glycol | 0.45 |
| N-methyl pyrrolidone | >0.50 |
| dibutyl ether | 0.125 |
| ethanol | 0.325 |

Methyl benzyl dicyanodiamide also was tested according to the same procedure. Less than 0.50 g was dissolved in acetone and methyl ethyl ketone but 0.15 g was dissolved in propylene glycol methyl ether.

EXAMPLE 8

Solubility testing for substituted cyanoguanidines (dicyanodiamide) was conducted by using a weight ratio of the compound to solvent of 1:10. The sample was agitated slightly and dissolution recorded at 25 °C; complete dissolution receives a rating of +, partial dissolution is +δ, and no dissolution is rated as -. The sample was then heated to 50 °C for 30 minutes and the solubility recorded using the same rating system.

TABLE B

| Experimental Solubility for Substituted Benzyl-Dicyanodiamides | | | | | | | | | | |
|---|---|---|---|---|---|---|---|---|---|---|
| Solvent | Substituted Benzyl-Dicyanodiamide | | | | | | | | | |
| | BZDICY[a] | | MBZDICY[b] | | MOBZDICY[c] | | DMABZDICY[d] | | HBZDICY[e] | |
| Temperature (°C) | 25 | 50 | 25 | 50 | 25 | 50 | 25 | 50 | 25 | 50 |
| Acetone | + | + | - | + | + | + | - | + | +δ | +δ |
| Methyl ethyl ketone(MEK) | + | + | - | + | + | + | - | + | +δ | +δ |
| 1-Methoxy-2-Propanol | + | + | + | + | + | + | - | + | + | + |
| MEK/1-methoxy-2-propanol (50:50) | + | + | - | + | + | + | - | + | + | + |
| N,N-Dimethylformamide (DMF) | + | + | + | + | + | + | + | + | + | + |
| Dimethyl sulfoxide (DMSO) | + | + | + | + | + | + | + | + | + | + |
| N,N-Dimethylacetamide (DMAC) | + | + | + | + | + | + | - | - | + | + |
| N-Methyl-2-pyrrolidinone (NMP) | + | + | + | + | + | + | + | + | + | + |
| Ethylacetate | - | + | - | - | - | + | - | - | - | - |
| Methanol | + | + | - | + | + | + | - | - | + | + |
| Ethanol | + | + | - | + | + | + | - | - | + | + |
| Toluene | - | - | - | - | - | - | - | - | - | - |

(a) benzyl dicyanodiamide
(b) 4-methyl-benzyl dicyanodiamide
(c) 4-methoxy benzyl dicyanodiamide
(d) 4-(dimethylamino)benzyl dicyanodiamide
(e) 4-hydroxy benzyl dicyanodiamide

Table B shows that compounds which contain a benzyl group attached to the amino hydrogens provide significantly improved solubility to the cyanoguanidine. The best solubility is seen with benzyl substituted cyanoguanidine (BZDICY) and a methoxy-substituted benzyl compound (MOBZDICY). Other related compounds are seen to have improved solubility but are somewhat less soluble.

EXAMPLE 9

Part A: 3.10 g of BZDICY was dissolved in 5.60 g of 1-methoxy-2-propanol and heated to 50°C for 30 minutes with stirring. 0.050 g of 2-Methyl-imidazole (2MI) and 6.92 g of methyl ethyl ketone (MEK) was added to the above solution with stirring.

Part B: 2.0 g of acetone was added to 50.0 g of Dow epoxy 71808 resin (diglycidyl Bisphenol-A (DGEBA) and brominated DGEBA) and mixed well.

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa. The polymer had the following properties as a function of cure.

| Time (min.) | Tg (°C) |
|---|---|
| 0 | 48 |
| 10 | 78 |
| 20 | 80 |
| 30 | 90 |
| 60 | 93 |
| 90 | 99 |
| 120 | 101 |
| 150 | 104 |
| 180 | 115 |
| 360 | 126 |

The final polymer properties after 360 minutes of cure at 170°C: Tg by differential scanning calorimeter (DSC) = 126°C, Tg by thermomechanical analysis (TMA) = 117±5°C, $\alpha_g$ = 46±3 ppm/°C, $\alpha_{180}$ = 104±4 ppm/°C.

EXAMPLE 10

Part A: 3.10 g of BZDICY was dissolved in 5.60 g of 1-methoxy-2-propanol and heated to 50°C for 30 minutes with stirring. 0.050 g of Benzyldimethylamine (BDMA) and 6.92 g of methyl ethyl ketone (MEK) was added to the above solution with stirring.

Part B: 2.0 g of acetone was added to 50.0 g of Dow epoxy 71808 resin (diglycidyl Bisphenol-A (DGEBA) and brominated DGEBA) and mixed well.

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.
The polymer had the following properties as a function of cure.

| Time (min.) | Tg (°C) |
|---|---|
| 0 | 44 |
| 10 | 78 |
| 20 | 79 |
| 30 | 80 |
| 60 | 91 |
| 90 | 96 |
| 120 | 97 |
| 150 | 97 |
| 180 | 101 |
| 360 | 116 |

The final polymer properties after 360 minutes of cure at 170°C: Tg (DSC) = 116°C, Tg (TMA) = 108±9°C, $\alpha_g$ = 46±7 ppm/°C, $\alpha_{180}$ = 111±2 ppm/°C.

EXAMPLE 11

Comparative

Part A: 1.12 g of DICY was dissolved in 6.92 g of Dimethylformamide (DMF) and 5.60 g of 1-methoxy-2-propanol and heated to 50°C for 30 minutes with stirring. 0.050 g of 2-Methyl-imidazole (2MI) was added to the above solution with stirring.

Part B: 2.0 g of acetone was added to 50.0 g of Dow epoxy 71808 resin (diglycidyl Bisphenol-A (DGEBA) and brominated DGEBA) and mixed well.

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 47 |
| 10 | 80 |
| 20 | 80 |
| 30 | 94 |
| 60 | 95 |
| 90 | 111 |
| 120 | 119 |
| 150 | 120 |
| 180 | 125 |
| 360 | 128 |

The final polymer properties after 360 minutes of cure at 170°C: Tg (DSC) = 128°C, Tg (TMA) = 116 ± 5°C, $\alpha_g$ = 46 ± 9 ppm/°C, $\alpha_{180}$ = 100 ± 5 ppm/°C.

EXAMPLE 12

Comparative

Part A: 1.12 g of DICY was dissolved in 5.20 g of 1-methoxy-2-propanol and 6.92 g of DMF heated to 50°C for 30 minutes with stirring. 0.044 g of 2-Methyl-imidazole (2MI) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 44 |
| 10 | 80 |
| 20 | 83 |
| 30 | 70 |
| 60 | 83 |
| 90 | 110 |
| 120 | 114 |
| 150 | 116 |
| 180 | 126 |
| 360 | 130 |

The final polymer properties after 360 minutes of cure at 170°C: Tg (DSC) = 130°C, Tg (TMA) = 114.8 ± 4.3°C, $\alpha_g$ = 34.6 ± 6.6 ppm/°C, $\alpha_{180}$ = 91.1 ± 5.0 ppm/°C.

EXAMPLE 13

Comparative

Part A: 2.09 g of DICY was dissolved in 5.20 g of 1-methoxy-2-propanol and 6.92 g of DMF heated to 50°C for 30 minutes with stirring. 0.044 g of 2-Methyl-imidazole (2MI) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured

at 170 ° C in a hydraulic press at 1379 kPa.
The polymer had the following properties as a function of cure.

| Time (min.) | Tg (° C) |
|---|---|
| 0 | 46 |
| 10 | 65 |
| 20 | 66 |
| 30 | 80 |
| 60 | 78 |
| 90 | 94 |
| 120 | 98 |
| 150 | 111 |
| 180 | 118 |
| 360 | 118 |

The final polymer properties after 180 minutes of cure at 170 ° C: Tg (DSC) = 118 ° C, Tg (TMA) = 120±3 ° C, $\alpha_g$ = 33±7 ppm/ ° C, $\alpha_{180}$ = 117±6 ppm/ ° C.

## EXAMPLE 14

### Comparative

Part A: 1.67 g of DICY was dissolved in 5.20 g of 1-methoxy-2-propanol and 6.92 g of DMF heated to 50 ° C for 30 minutes with stirring. 0.044 g of 2-Methyl-imidazole (2MI) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170 ° C in a hydraulic press at 1379 kPa.
The polymer had the following properties as a function of cure.

| Time (min.) | Tg (° C) |
|---|---|
| 0 | --- |
| 10 | 62 |
| 20 | 69 |
| 30 | 81 |
| 60 | 88 |
| 90 | 93 |
| 120 | 98 |
| 150 | 103 |
| 180 | 110 |
| 360 | 113 |

The final polymer properties after 360 minutes of cure at 170 ° C: Tg (DSC) = 113 ° C, Tg (TMA) = 124 ± 5 ° C, $\alpha_g$ = 40 ± 3 ppm/ ° C, $\alpha_{180}$ = 91 ± 4 ppm/ ° C.

## EXAMPLE 15

Part A: 3.08 g of BZDICY was dissolved in 5.20 g of 1-methoxy-2-propanol and heated to 50 ° C for 30 minutes with stirring. 0.044 g of 2-Methyl-imidazole (2MI) and 6.92 g of methyl ethyl ketone (MEK) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170 ° C in a hydraulic press at 1379 kPa.

The polymer had the following properties as a function of cure.

| Time (min.) | Tg (°C) |
|---|---|
| 0 | 40 |
| 10 | 82 |
| 20 | 87 |
| 30 | 94 |
| 60 | 94 |
| 90 | 90 |
| 120 | 95 |
| 150 | 100 |
| 180 | 100 |
| 360 | 101 |

The final polymer properties after 360 minutes of cure at 170°C: Tg (DSC) = 101°C, Tg (TMA) = 102 ± 5 °C, $\alpha_g$ = 34 ± 3 ppm/°C, $\alpha_{180}$ = 102 ± 4 ppm/°C.

EXAMPLE 16

Part A: 3.34 g of MBZDICY was dissolved in 5.20 g of 1-methoxy-2-propanol and heated to 50°C for 30 minutes with stirring. 0.044 g of 2-Methyl-imidazole (2MI) and 6.92 g of MEK was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 33 |
| 10 | 89 |
| 20 | 91 |
| 30 | 93 |
| 60 | 103 |
| 90 | 105 |
| 120 | 106 |
| 150 | 112 |
| 180 | 115 |
| 360 | 126 |

The final polymer properties after 360 minutes of cure at 170°C: Tg (DSC) = 126°C, Tg (TMA) = 118±6°C, $\alpha_g$ = 27±2 ppm/°C, $\alpha_{180}$ = 105±2 ppm/°C.

EXAMPLE 17

Part A: 5.78 g of BZDICY was dissolved in 5.20 g of 1-methoxy-2-propanol and heated to 50°C for 30 minutes with stirring. 0.044 g of 2-Methyl-imidazole (2MI) and 6.92 g of methyl ethyl ketone (MEK) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 43 |
| 10 | 87 |
| 20 | 89 |
| 30 | 85 |
| 60 | 87 |
| 90 | 97 |
| 120 | 101 |
| 150 | 103 |
| 180 | 102 |
| 360 | 113 |

The final polymer properties after 360 minutes of cure at 170°C: Tg (DSC) = 113°C, Tg (TMA) = 102 ± 6°C, $\alpha_g$ = 47 ± 4 ppm/°C, $\alpha_{180}$ = 108 ± 4 ppm/°C.

EXAMPLE 18

Part A: 4.62 g of BZDICY was dissolved in 5.20 g of 1-methoxy-2-propanol and heated to 50°C for 30 minutes with stirring. 0.044 g of 2-Methyl-imidazole (2MI) and 6.92 g of MEK was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 41 |
| 10 | 76 |
| 20 | 82 |
| 30 | 81 |
| 60 | 90 |
| 90 | 98 |
| 120 | 100 |
| 150 | 107 |
| 180 | 109 |
| 360 | 113 |

The final polymer properties after 360 minutes of cure at 170°C: Tg (DSC) = 113°C.

EXAMPLE 19

Part A: 3.10 g of BZDICY was dissolved in 5.60 g of 1-methoxy-2-propanol and heated to 50°C for 30 minutes with stirring. 0.044 g of Benzyldimethylamine (BDMA) and 6.92 g of methyl ethyl ketone (MEK) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 31 |
| 30 | 60 |
| 60 | 98 |
| 90 | 108 |
| 120 | 112 |
| 150 | 117 |
| 180 | 124 |
| 360 | 122 |

The final polymer properties after 360 minutes of cure at 170°C: Tg (DSC) = 122°C, Tg (TMA) = 103 ± 3 °C, $\alpha_g$ = 39 ± 5 ppm/°C, $\alpha_{180}$ = 102 ± 4 ppm/°C.

EXAMPLE 20

Part A: 3.10 g of BZDICY was dissolved in 5.60 g of 1-methoxy-2-propanol and heated to 50°C for 30 minutes with stirring. 0.044 g of 4-(dimethylamino)-N,N-dimethylbenzylamine (DMBDMA) and 6.92 g of MEK was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 25 |
| 30 | 46 |
| 60 | 89 |
| 90 | 111 |
| 120 | 113 |
| 150 | 116 |
| 180 | 117 |
| 360 | 117 |

The final polymer properties after 360 minutes of cure at 170°C: Tg (DSC) = 117°C, Tg (TMA) = 104 ± 3 °C, $\alpha_g$ = 46.95 ± 2.7 ppm/°C, $\alpha_{180}$ = 107.9 ± 2.2 ppm/°C.

EXAMPLE 21

Part A: 3.62 g of MOBZDICY was dissolved in 5.20 g of 1-methoxy-2-propanol and heated to 50°C for 30 minutes with stirring. 0.044 g of 2-Methyl-imidazole (2MI) and 6.92 g of methyl ethyl ketone (MEK) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 43 |
| 10 | 86 |
| 20 | 87 |
| 30 | 91 |
| 60 | 90 |
| 90 | 106 |
| 120 | 107 |
| 150 | 109 |
| 180 | 115 |
| 360 | 125 |

The final polymer properties after 360 minutes of cure at 170°C: Tg (DSC) = 125°C, Tg (TMA) = 109 ± 3°C, $\alpha_g$ = 39 ± 8 ppm/°C, $\alpha_{180}$ = 100 ± 3 ppm/°C.

EXAMPLE 22

Part A: 3.86 g of DMABZDICY was dissolved in 5.20 g of 1-methoxy-2-propanol and heated to 50°C for 30 minutes with stirring. Then 6.92 g of MEK was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.
The polymer had the following properties as a function of cure.

| Time (min.) | Tg (°C) |
|---|---|
| 0 | 54 |
| 30 | 86 |
| 60 | 93 |
| 90 | 108 |
| 120 | 110 |
| 150 | 114 |
| 180 | 119 |
| 360 | 125 |

The final polymer properties after 360 minutes of cure at 170°C: Tg (DSC) = 125°C, Tg (TMA) = 104±5°C, $\alpha_g$ = 45±7 ppm/°C, $\alpha_{180}$ = 104±2 ppm/°C.

EXAMPLE 23

Part A: 3.86 g of DMABZDICY was dissolved in 5.20 g of 1-methoxy-2-propanol and heated to 50°C for 30 minutes with stirring. 0.044 g of 2-Methyl-imidazole (2MI) and 6.92 g of methyl ethyl ketone (MEK) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.
The polymer had the following properties as a function of cure.

| Time (min.) | Tg (°C) |
|---|---|
| 0 | 46 |
| 30 | 93 |
| 60 | 98 |
| 90 | 110 |
| 120 | 114 |
| 150 | 120 |
| 180 | 120 |
| 360 | 125 |

The final polymer properties after 360 minutes of cure at 170°C: Tg (DSC) = 125°C, Tg (TMA) = 128±7°C, $\alpha_g$ = 42±9 ppm/°C, $\alpha_{180}$ = 126±15 ppm/°C.

EXAMPLE 24

Part A: 4.60 g of BZDICY was dissolved in 5.60 g of 1-methoxy-2-propanol and heated to 50°C for 30 minutes with stirring. 0.090 g of 1-(2-cyanoethyl)-2-ethyl-4-methylimidazole (CEM) was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 23 |
| 10 | 80 |
| 20 | 90 |
| 30 | 90 |
| 60 | 96 |
| 90 | 100 |
| 120 | 110 |
| 150 | 115 |
| 180 | 116 |
| 360 | 120 |

The final polymer properties after 360 minutes of cure at 170°C: Tg (DSC) = 120°C, Tg (TMA) = 122 ± 2°C, $\alpha_g$ = 34 ± 5 ppm/°C, $\alpha_{180}$ = 92 ± 5 ppm/°C.

EXAMPLE 25

Part A: 2.40 g of BZDICY was dissolved in 12.60 g of 1-methoxy-2-propanol and heated to 50°C for 30 minutes with stirring. 0.15 g of 2-Methyl-imidazole (2MI) and 15.50 g of methyl ethyl ketone (MEK) was added to the above solution with stirring.

Part B: 69.50 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 36 |
| 30 | 80 |
| 60 | 84 |
| 90 | 107 |
| 120 | 117 |
| 150 | 119 |
| 180 | 124 |
| 360 | 129 |

The final polymer properties after 360 minutes of cure at 170°C: Tg (DSC) = 129°C, Tg (TMA) = 102±2°C, $\alpha_g$ = 42±11 ppm/°C, $\alpha_{180}$ = 107±7 ppm/°C.

EXAMPLE 26

Part A: 3.38 g of HBZDICY was dissolved in 5.20 g of 1-methoxy-2-propanol and heated to 50°C for 30 minutes with stirring. 0.044 g of 4-(dimethylamino)-N,N-dimethylbenzylamine (DMBDMA) and 6.92 g of MEK was added to the above solution with stirring.

Part B: 50.0 g of Dow epoxy 71881 resin (diglycidyl Bisphenol-A (DGEBA) and brominated DGEBA).

Part A and Part B were mixed together and allowed to age for 24 hours. The resin varnish was then B-Staged on a hot plate in a thin casting pan. The B-Staged resin was ground into fine powder and then cured at 170°C in a hydraulic press at 1379 kPa.

The polymer had the following properties as a function of cure.

| Time (min.) | Tg (°C) |
|---|---|
| 0 | 34 |
| 30 | 90 |
| 60 | 82 |
| 90 | 113 |
| 120 | 120 |
| 150 | 121 |
| 180 | 123 |
| 360 | 127 |

The final polymer properties after 360 minutes of cure at 170°C: Tg (DSC) = 127°C, Tg (TMA) = 112 ± 1°C, $\alpha_g$ = 53 ± 4 ppm/°C, $\alpha_{180}$ = 126 ± 6 ppm/°C.

EXAMPLE 27

Two substituted cyanoguanidines, N-Benzyl-dicyanodiamide (BZDICY and N-(4-Methylbenzyl)-dicyanodiamide (MBZDICY), were used to prepare epoxy laminates. Four accelerators were tested for each of the two cyanoguanidines. Formulations using Dow 71881 epoxy resins were compounded with 7.75 parts of BZDICY for 100 parts of epoxy or 8.37 parts of MBZDICY per 100 parts of epoxy. The BZDICY and MBZDICY were dissolved in a solvent mixture of 4 parts propylene glycol metyl ether and 5 parts MEK. The accelerators were added until a 150 second gel time was obtained when a sample was heated on a hot plate at 171°C. Laminates were then prepared using the resin formulations by coating the resins onto type 1080-307 glass fabric (supplied by Clark-Schwebel). Samples of laminates 305 mm x 305 mm were made using 2 piles of the resin coated fabric. These were converted to a prepreg by curing in hot air for 2 to 8 minutes at 177°C. Final curing to a finished laminate was carried out by applying pressure of 2068 kPa (gauge) during a heating cycle including a rise up to 176.6°C, maintaining at 176.6°C for 50 minutes, and then cooling to 20°C over 15 minutes. The resulting laminates were tested for properties related to their performance as electronic circuit boards and compared with similar laminates made using dicyanodiamide (cyanoguanidine). The results of these tests are summarized in the following table.

Table E

| | Control A(1) | Control B(2) | BZDICY Accelerators | | | | MBZDICY Accelerators | | | |
|---|---|---|---|---|---|---|---|---|---|---|
| | | | CDI(3) | DMAP(4) | 2MI(5) | 2PI(6) | CDI | DMAP | 2MI | 2PI |
| Water Absorption, wt.% (7) | 2.76 | 2.82 | 3.08 | 3.29 | 2.82 | 2.5 | 2.39 | - | 2.44 | 3.04 |
| Arc Resistance, sec (8) | N/A | 84 | 75 | 73 | N/A | 69 | 86 | - | 64 | 81 |
| $H_2SO_4$ Etchability wt.%(9) | 9.35 | 12 | 12.9 | 12.3 | 14 | 9.12 | - | - | - | |
| $MeCL_2$ Absorption wt. % (10) | 12.7 | 7.79 | 13.87 | 15.11 | 13.14 | 12.36 | 9.84 | - | 11 | 11 |
| Tg-DSC(11), °C | 131 | 135 | 124 | 125 | 125 | 125 | 124 | 123 | 124 | 125 |
| Peel Strength A(12) | 57.9/73.8 | 69.6/71.7 | 71.7/ 75.2 | 73.8/ 71.7 | 75.8/ 77.2 | 66.9/ 59.3 | 75.8/ 71.7 | 71.0/ 75.2 | 72.3/ 75.8 | 75.8/ 75.8 |
| Peel Strength E(13) | 57.9/66.2 | 63.4/61.4 | 65.5/ 66.2 | 62/ 62 | 59.3/ 58.6 | 45.5/ 45.5 | 82/ 84.1 | 54.5/ 68.9 | 50.3/ 55.8 | 64.1/ 68.9 |
| Solder Blister sec (14) | 43 | 21 | 44 | 18 | 74 | 54 | 75 | 13 | 83 | 77 |

(1) 100 parts Dow 2483, 2.8 parts dicyanodiamide, 0.08 parts 2-methyl-imidazole

(2) 100 parts Dow 71881, 2.8 parts dicyanodiamide, 0.08 parts 2-methyl-imidazole

(3) 1,1'-carbonyldiamidzole

(4) 2-Dimethylaminopyridine

(5) 2-methyl-imidazole

(6) 2-phenyl-imidazole

(7) 24 hours in boiling water (100°C)

(8) seconds to failure after conditioning for 48 hours in 50°C water (ASTM 2.5.1)

(9) percent weight loss after 30 seconds in concentrated $H_2SO_4$ at room temperature

(10) percent weight gain after 20 minute submersion at room temperature

(11) glass transition temperature by differential scanning calorimeter method

(12) kPa at room temperature

(13) kPa after 1 hour at 125°C

(14) time to failure in 550°C solder bath

From the above results it was concluded that 2-methyl-imidazole and 1,1'-carbonyldiamidazole were preferred of the accelerators tested. Both BZDICY and MBZDICY were considered to perform as well as dicyanodiamide in the Control samples and since the substituted cyanoguanidines permit the use of more acceptable solvents, they have an important advantage over dicyanodiamide (cyanoguanidine).

## Claims

1. A composition comprising an epoxy resin and a substituted cyanoguanidine curing agent for said epoxy resin characterized in that said substituted cyanoguanidine has the formula

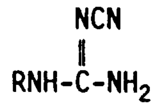

where R is $-CH_2C_6H_4X$, or $-CH_2CH_2C_6H_4X$,
and X is either -H, $CH_3$, $-OCH_3$, -OH, or $-NY_2$
and Y is -H or $-CH_3$

2. A method for curing epoxy resins comprising combining said epoxy resin with an effective amount of a substituted cyanoguanidine having the formula

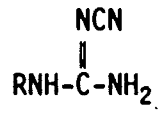

where R is $-CH_2C_6H_4X$, or $-CH_2CH_2C_6H_4X$,
and X is either -H, $-CH_3$, $-OCH_3$, -OH, or $-NY_2$
and Y is -H or $-CH_3$

3. The method of Claim 2 wherein said substituted cyanoguanidine is present in amounts up to 20 weight percent.

**Patentansprüche**

1. Zusammensetzung mit einem Epoxidharz und einem substituierten Cyanoguanidin als Härtungsmittel für das Epoxidharz, dadurch gekennzeichnet, daß das substituierte Cyanoguanidin die Formel

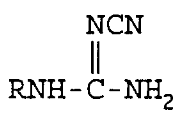

besitzt, worin R $-CH_2C_6H_4X$ oder $-CH_2CH_2C_6H_4X$ ist und
X entweder -H, $-CH_3$, $-OCH_3$, OH oder $NY_2$ ist
und Y gleich -H oder $-CH_3$ ist.

2. Verfahren zum Härten von Epoxidharzen, welches das Vermischen des Epoxidharzes mit einer wirksamen Menge eines substituierten Cyanoguanidins mit der Formel

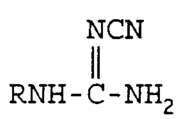

umfaßt, worin R $-CH_2C_6H_4X$ oder $-CH_2CH_2C_6H_4X$ ist und
X entweder -H, $-CH_3$, $-OCH_3$, OH oder $NY_2$ ist
und Y gleich -H oder $-CH_3$ ist.

3. Verfahren nach Anspruch 2, bei dem das substituierte Cyanoguanidin in Mengen bis zu 20 Gewichtsprozent vorhanden ist.

## Revendications

**1.** Composition comprenant une résine époxy et un agent de polymérisation de cyanoguanidine substituée pour ladite résine époxy, caractérisée en ce que ladite cyanoguanidine substituée répond à la formule:

$$\begin{array}{c} NCN \\ \| \\ RNH-C-NH_2 \end{array}$$

dans laquelle R est $-CH_2C_6H_4X$ ou $-CH_2CH_2C_6H_4X$,
et X est soit -H, $-CH_3$, $-OCH_3$, -OH,
soit $-NY_2$
et Y est -H ou $-CH_3$

**2.** Procédé pour polymériser des résines époxy comprenant la combinaison de ladite résine époxy avec une quantité efficace d'une cyanoguanidine substituée ayant la formule :

$$\begin{array}{c} NCN \\ \| \\ RNH-C-NH_2 \end{array}$$

dans laquelle R est $-CH_2C_6H_4X$ ou $-CH_2CH_2C_6H_4X$,
et X est soit -H, $-CH_3$, $-OCH_3$, -OH,
soit $-NY_2$
et Y est -H ou $-CH_3$.

**3.** Procédé selon la revendication 2, dans lequel ladite cyanoguanidine substituée est présente dans des quantités atteignant 20 pour cent en poids.